# EUROPEAN PATENT APPLICATION

(11) **EP 2 532 341 A1**
(43) Date of publication of application: **12.12.2012**
(21) Application number: 11004612.5
(22) Date of filing: 07.06.2011
(51) Int. Cl.: A61H 33/14, A61M 31/00, A61M 35/00

(54) **Kit of parts for the treatment of infectious disease**

(71) Applicant: Krebber, Burghardt, 86899 Landsberg/Lech (DE)
(72) Inventor: Krebber, Burghardt, 86899 Landsberg/Lech (DE)
(74) Representative: Blodig, Wolfgang

(57) **Abstract**

Kit of parts comprising:
(a) an ozone generator for generating an ozone containing gas;
(b) a first applicator comprising
■ a surface for providing an treatment chamber with the skin of a patient;
■ an inlet orifice for establishing a fluid flow communication with the ozone generator;
■ an opening having a sealing edge for introducing the body of the patient or a part thereof and sealing the treatment chamber;

(c) a second applicator for rectally introducing ozone containing gas into a patient, said applicator comprising
■ a hollow elongate body having a proximal end and a distal end;
■ an inlet orifice provided at the proximal end for establishing a fluid flow communication with the ozone generator;
■ an outlet orifice provided at the distal end, which is in fluid flow communication with the inlet orifice.

## Description

### Field of the Invention

The present invention relates to a kit of parts for the treatment of an infectious disease by an ozone containing gas. More specifically, the kit of parts of the present invention may be used for the treatment of food-borne illness. Moreover, the present invention relates to an ozone containing gas for use in the prevention or treatment of food-born illness. Finally, the present invention also relates to a method of treatment of a food-born illness. The food-borne illness may include E. *coli* 0157:H7 infections leading to haemolytic-uremic syndrome.

### Background of the Invention

Haemolytic-uremic syndrome (HUS) is a disease characterized by haemolytic anaemia, acute renal failure (uraemia) and a low platelet count (thrombocytopenia). Most cases are preceded by an episode of diarrhea, which is acquired as a food-borne illness. It is a medical emergency and carries a 5―10% mortality, whereby of the remainder, a significant proportion may develop chronic kidney disease and become reliant on renal replacement therapy.

As haemolytic-uremic syndrome is a life-threatening condition, it is usually treated in an intensive care unit. Blood transfusions and kidney dialysis are often required.

Antibiotic treatment of E. coli O157:H colitis may stimulate further verotoxin production and thereby increase the risk of HUS. Therefore, treatment of HUS is generally supportive. Untreated HUS in adults, however, may progress to end-stage organ damage. Platelet transfusion may actually worsen the outcome.

Therefore, there is a need for a treatment of a food-borne illness such as HUS.

### Summary of the Invention

It is the problem of the present invention to provide a kit of parts for the treatment of an infectious disease.

It is a further problem of the present invention to a method of treatment of a food-born illness, which may include E. *coli* O157:H infections leading to hemolytic-uremic syndrome.

This problem is solved according to the present invention as defined by the claims. Specifically, the present invention provides a kit of parts comprising:
(a) an ozone generator for generating an ozone containing gas;
(b) a first applicator comprising
   - a surface for providing an treatment chamber with the skin of a patient;
   - an inlet orifice for establishing a fluid flow communication with the ozone generator;
   - an opening having a sealing edge for introducing the body of the patient or a part thereof and sealing the treatment chamber;
(c) a second applicator for rectally introducing ozone containing gas into a patient, said applicator comprising
   - a hollow elongated body having a proximal end and a distal end;
   - an inlet orifice provided at the proximal end for establishing a fluid flow communication with the ozone generator;
   - an outlet orifice provided at the distal end, which is in fluid flow communication with the inlet orifice.

Moreover, the present invention provides an ozone-containing gas for use in the treatment of food-born illness by a method comprising
(i) introducing rectally the ozone-containing gas into the intestine of a patient for topical administration of ozone to the intestine and
(ii) applying ozone containing gas to the skin of a patient for systemic treatment of the patient with ozone.

Finally, the present invention provides a method of treatment of a food-born illness, said method comprising
- introducing rectally an ozone-containing gas into the intestine of a patient for topical administration of ozone to the intestine wall and
- applying ozone containing gas to the skin of a patient for systemic treatment of the patient with ozone.

The present invention is based on the recognition that food-borne illness such as HUS, may be prevented or treated by topically applying an ozone containing gas to the intestinal mucosa and by systemically applying an ozone containing gas to the patient by exposing the skin of the patient to an ozone containing gas.

When topically applying an ozone containing gas to the intestinal mucosa, bacterial growth adhering to the intestinal mucosa may be efficiently and safely controlled. When systemically applying an ozone containing gas to the patient by exposing the skin of the patient to an ozone containing gas, the ozone may diffuse through the skin and into the blood of the patient for systemic control of bacterial infection.

The present invention is further based on the recognition that a continuous flow of ozone may be topically applied to the intestinal mucosa and systemically applied by exposing the skin of the patient to the ozone containing gas applied over an extended period of time without toxic side effects.

The present invention may use a partial pressure of ozone in the ozone containing gas which forces the ozone deep into the mucosal tissue and through the skin whereby an efficient treatment of food borne illness is possible without the need for additional antibiotic therapy. Accordingly, it is possible to efficiently interrupt infection of the patient due to bacterial growth in the patient while at the same time avoiding the risk that the bacteria develop resistance against the treatment.

### Detailed Description of the Preferred Embodiments

The kit of parts of the present invention comprises an ozone generator for generating an ozone containing gas.

The ozone generator may be a conventional ozone generator using an electric discharge for cleaving oxygen molecules in a plasma. Preferably, the ozone generator allows controlling the rate of ozone generation in order to adjust the ozone concentration in the ozone containing gas. The ozone containing gas generated by the ozone generator preferably has an ozone concentration of from 50 to 20.000 ppm, more preferably an ozone concentration of from 100 to 10.000 ppm.

The kit of parts according to the present invention may further comprise a first pump. Accordingly, the ozone generator according to the present invention may be used with the first pump being in fluid flow communication with the ozone generator for generating a flow of ozone containing gas. Preferably, the first pump is arranged so as to discharge a stream of gas, such as air, into the ozone generator for providing a continuous flow of ozone containing gas downstream from the first pump and the ozone generator. The gas may be air having an oxygen content in the range of from 20 to 99 percent by volume. Accordingly, pure air may preferably be used or a mixture comprising air and additional pure oxygen gas.

The first pump has a first throughput. Moreover, the first pump has a first pumping speed. Preferably, the first pump has a pumping speed in the range of from 1 to 10 l/min, more preferably from 2 to 6 I/min.

For safety reasons, the ozone generator is preferably arranged in series with the first and optionally a second pump downstream of the ozone generator which is in fluid flow communication with an applicator and which is adapted for removing any excess ozone containing gas from the applicator. The pumps and the ozone generator are preferably connected so that the ozone generator may only be activated when the pumps are running, thereby preventing an uncontrolled discharge of ozone containing gas from the ozone generator.

Accordingly, the kit of parts according to the present invention may further comprise a second pump. The second pump which may be brought in fluid flow communication with the outlet orifice of an applicator for removing gas has a second throughput. The ratio of the second throughput to the first throughput is preferably at least 1.5 : 1, preferably 2:1. Moreover, the second pump preferably has a pumping speed in the range of from 1.5 to 15 l/min, more preferably in the range of from 2 to 10 l/min. Preferably, the activation of the second pump is a necessary condition for the possibility of activating the ozone generator so that the contamination of the patient may be excluded.

The kit of parts of the present invention may further comprise an ozone scavenging means which is in fluid communication with the outlet orifice of the applicator. The scavenging means may contain a reducing agent which eliminates any ozone remaining in the flow of ozone containing gas discharged from the treatment chamber before entering the second pump.

Moreover, the device of the present invention may further comprise a moisture trapping means which is in fluid communication with the outlet orifice of the applicator. The moisture trapping means may contain a drying agent or a cold surface for eliminating any water vapours remaining in the flow of ozone containing gas discharged from the treatment chamber before entering the second pump.

The kit of parts of the present invention further comprises a first applicator comprising a surface for providing an treatment chamber with the skin of a patient, an inlet orifice for establishing a fluid flow communication with the ozone generator, an opening having a sealing edge for introducing the body of the patient or a part thereof and sealing the treatment chamber.

The first applicator may further comprise an outlet orifice which may be connected in fluid flow communication with the second pump. The first applicator may preferably be made of a flexible resin film. A suitable flexible film may be selected from polyethylene films, polypropylene films, or copolymers of ethylene and propylene. The first applicator may contain structural elements for maintaining a treatment chamber even in case of a negative pressure within the treatment chamber desirable for safety reasons according to the present invention.

Preferably, the first applicator has a bag shape of sufficient size for accommodating the legs and trunk of the patient. The bag-shaped applicator has an opening which comprises a sealing edge for sealing the treatment chamber around the chest of a patient. The sealing edge may seal the treatment chamber by using an adhesive tape or by tightening a rope which is provided along the edge of the bag-shaped applicator.

The systemic treatment of the patient by using the first applicator of the present invention includes the exposure of the skin surface of the patient to ozone molecules in a concentration suitable for controlling bacterial growth in the blood of the patient. The treatment includes the penetration of the skin surface by the ozone molecules and distribution of the ozone molecules or reaction products thereof throughout the body of the patient so that bacterial growth may be systemically controlled. For this purpose, a substantial portion of the total skin surface of the patient is exposed to a high ozone partial pressure over a prolonged period of time.

The kit of parts of the present invention further comprises a second applicator for rectally introducing ozone containing gas into a patient, said applicator comprising a hollow elongate body having a proximal end and a distal end, an inlet orifice provided at the proximal end for establishing a fluid flow communication with the ozone generator, and an outlet orifice provided at the distal end, which is in fluid flow communication with the inlet orifice.

The second applicator may be an apparatus including a barrel configured to be inserted into an anal canal. The barrel may have ends defining at least one first gas flow path into and out of the body. Preferably, the barrel is flexible so as to allow a painless insertion of the barrel into the intestine of the patient. The barrel may be made of a flexible tubing which is disposed of after the treatment.

Topical treatment of intestinal mucosa within the meaning of the present invention includes the exposure of intestinal surfaces to ozone molecules in a concentration suitable for controlling bacterial growth. Preferably, the treatment also includes the penetration of the intestinal surface by the ozone molecules so that bacterial growth below the surface of the intestinal mucosa may be controlled. Since anaerobic bacteria propagate in an environment where oxygen is not available, the treatment according to the present invention may be directed to providing an exposure of such bacteria even in such anaerobic environments. For this purpose, the intestinal tissue is exposed to a high ozone partial pressure over a prolonged period of time. Accordingly, it is possible to not only treat and prevent food borne illness close to the surface of the intestinal mucosa, but also to control bacteria food borne illness in layers below the intestinal mucosa.

The ozone generator according to the present invention may be used as follows according to a preferred embodiment. Prior to activating the ozone generator, the applicator is placed in or around the patients so that a treating chamber is formed by either by the inner surface of the first applicator and the skin to be treated, or by the intestinal mucosa to be treated. Subsequently, a gas flow may be initiated by activating the first pump and preferably the second pump. Provided that the gas flow is established, the ozone generator may be activated. Accordingly, the flow of gas will then include an amount of ozone to which the surface inside the treating chamber is exposed. The treatment of a intestinal mucosa of a patient by applying a ozone may be carried out for a total period of time per day in the range of from 30 seconds to 600 minutes, preferably from 1 to 60 minutes. The treatment may preferably be carried out once a day to five times, preferably 2 to 4 times a day for 1 to 7 day, preferably 3 to 5 days.

The present invention also provides an ozone-containing gas for use in the treatment or prevention of food-born illness. Preferably, the food-borne illness is HUS. In particular, the food-born illness may be hemolytic-uremic syndrome caused by the enterohemorrhagic E. coli Escherichia coli 0104:H4. The treatment or prevention of the food borne illness is a method comprising introducing rectally the ozone-containing gas into the intestine of a patient for topical administration of ozone to the intestinal mucosa and applying ozone containing gas to the skin of a patient for systemic treatment of the patient with ozone. The order of the rectal introduction the ozone-containing gas into the intestine of the patient for topical administration of ozone to the intestinal mucosa and the application of the ozone containing gas to the skin of a patient for systemic treatment of the patient with ozone is not specifically limited. It is possible to first topically apply the ozone containing gas according to the invention and subsequently systemically apply the ozone containing gas. In an alternative embodiment, the ozone containing gas is first systemically introduced and subsequently topically applied.

The rectal introduction of the ozone-containing gas into the intestine of a patient for topical administration of ozone to the intestinal mucosa may be carried out by using the second applicator.

The application of ozone containing gas to the skin of a patient for systemic treatment of the patient with ozone may be carried out by using the first applicator.

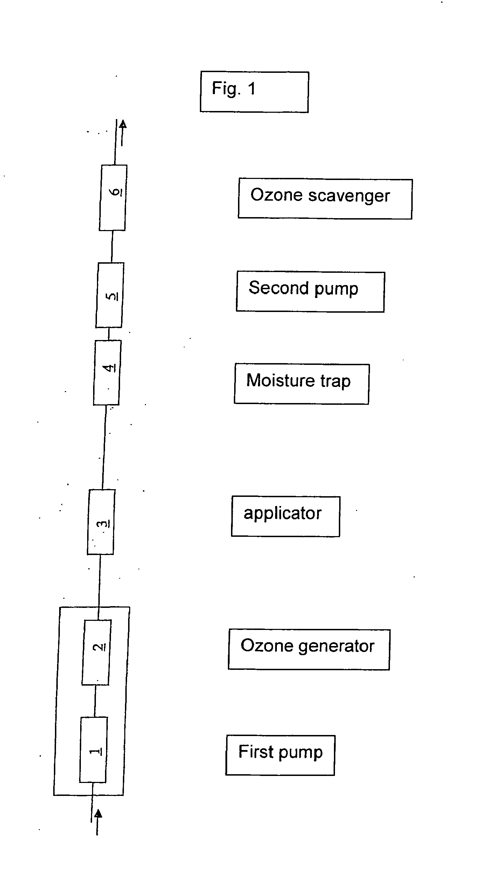

Figure 1 shows a schematic representation of a preferred configuration for using the kit of parts of the invention. A first pump 1 is fluid communication with an ozone generator. The first pump 1 and the ozone generator 2 may be combined in a single device for providing a flow of ozone gas. The ozone generator 2 is in fluid flow communication with the an applicator 3. The applicator is preferably the first applicator of the present invention. The applicator is in fluid flow communication with a ozone scavenging means 5 for eliminating excess ozone present in the flow of gas. The ozone scavenging means 5 is in fluid flow communication with a second pump 6 which is a suction pump. In the figure, an optional moisture trapping means 4 is also shown. The order of the moisture trapping means and the ozone scavenging means may be reversed.

Now the use of the first applicator will be described. The patient is introduced into the first applicator so that a substantial portion of the skin of the patient is covered by the applicator. Subsequently, the connectors are connected to tubing so that the inner wall of the first applicator forming a treatment chamber with the skin of the patient is connected to the ozone generator and the connector of the discharge channel is connected to the tubing for fluid flow communication with the second pump. Now the second pump is activated so that gas is removed by suction from the first applicator. Subsequently, the first pump and the ozone generator are activated whereby a flow of ozone containing gas enters the treatment chamber and exits through the outlet orifice of the second applicator. Since the through-put of the second pump is preferably in the same range or higher than the through-put of the first pump, ozone gas cannot escape from the first applicator.

Now the use of the second applicator will be described. The second applicator is rectally introduced into the patient so that a substantial portion of the intestinal mucosa of the patient may be treated according to the present invention. Subsequently, the connectors are connected to tubing so that the intestinal mucosa used the treatment chamber is connected to the ozone generator. Optionally, a discharge channel is established and connected to the tubing for fluid flow communication with the second pump. Subsequently, the first pump and the ozone generator are activated whereby a flow of ozone containing gas enters the treatment chamber.

The present invention also relates to the treatment of infections of a patient by human papillomavirus (HPV). HPV is a member of the papillomavirus family of viruses that is capable of infecting the stratified epithelium of the skin or mucous membranes. Since some types of HPV can cause warts, while others can lead to cancers of the cervix, vulva, vagina, and anus in women or cancers of the anus and penis in men, there is a high interest in the treatment of HPV infection.

Accordingly, the present invention also provides a kit of parts for the treatment of HPV infection. The kit of parts may include the ozone generator and the second applicator of the present invention, specifically an ozone generator for generating an ozone containing gas and a second applicator for vaginally introducing ozone containing gas into a female patient, said applicator comprising a hollow elongate body having a proximal end and a distal end, an inlet orifice provided at the proximal end for establishing a fluid flow communication with the ozone generator, and an outlet orifice provided at the distal end, which is in fluid flow communication with the inlet orifice.

Moreover, the present invention also provides ozone-containing gas for use in the treatment of HPV infection by a method comprising introducing vaginally the ozone-containing gas into the vagina of a female patient for topical administration of ozone to the vaginal mucosa.

Moreover, the present invention also provides a method for the treatment of HPV infection, said method comprising vaginally the ozone-containing gas into the vagina of a female patient for topical administration of ozone to the vaginal mucosa.

## Claims

1. Kit of parts comprising:
(a) an ozone generator for generating an ozone containing gas;
(b) a first applicator comprising
■ a surface for providing an treatment chamber with the skin of a patient;
■ an inlet orifice for establishing a fluid flow communication with the ozone generator;
■ an opening having a sealing edge for introducing the body of the patient or a part thereof and sealing the treatment chamber;
(c) a second applicator for rectally introducing ozone containing gas into a patient, said applicator comprising
■ a hollow elongate body having a proximal end and a distal end;
■ an inlet orifice provided at the proximal end for establishing a fluid flow communication with the ozone generator;
■ an outlet orifice provided at the distal end, which is in fluid flow communication with the inlet orifice.

2. The kit according to claim 1, which further comprises a first pump being in fluid flow communication with the ozone generator for generating a flow of ozone containing gas.

3. The kit according to claim 1 or 2, wherein the first applicator further comprises an outlet orifice.

4. The kit according to any one of the preceding claims, wherein the first applicator is made of a flexible resin film.

5. The kit according to claim 4, wherein the first applicator is a bag accommodating the legs and trunk of a patient whereby the opening has a sealing edge for sealing the treatment chamber around the chest of a patient.

6. The kit according to any one of the preceding claims, wherein the ozone containing gas generated by the ozone generator has an ozone concentration of from 50 to 20.000 ppm.

7. The kit according to any one of the preceding claims wherein the gas is air having an oxygen content in the range of from 20 to 99 percent by volume.

8. The device according to claim 7, wherein the first pump has a pumping speed in the range of from 1 to 10 l/min.

9. The kit according to claim 3, which further comprises a second pump which may be brought into fluid flow communication with the outlet orifice of the first applicator for removing gas from the treatment chamber.

10. Ozone-containing gas for use in the treatment or prevention of food-born illness by a method comprising
■ introducing rectally the ozone-containing gas into the intestine of a patient for topical administration of ozone to the intestinal mucosa and
■ applying ozone containing gas to the skin of a patient for systemic treatment of the patient with ozone.

11. The ozone containing gas use in the treatment of food-born illness according to claim 10, wherein the ozone containing gas has an ozone concentration of from 50 to 20.000 ppm.

12. The ozone containing gas use in the treatment of food-born illness according to claim 10 or 11, wherein the gas is air having an oxygen content in the range of from 20 to 99 percent by volume.

13. The ozone containing gas use in the treatment of food-born illness according to claim 10, wherein the illness is hemolytic-uremic syndrome caused by the enterohemorrhagic E. coli *Escherichia coli* 0104:H4.

14. Method of treatment of a food-born illness, said method comprising
■ introducing rectally an ozone-containing gas into the intestine of a patient for topical administration of ozone to the intestinal mucosa and
■ applying ozone containing gas to the skin of a patient for systemic treatment of the patient with ozone.

15. The method according to claim 14, wherein the food-born illness is hemolytic-uremic syndrome caused by the enterohemorrhagic E. coli *Escherichia coli* O104:H4.
